Europäisches Patentamt

European Patent Office

Office européen des brevets

(11) Publication number: **0 280 225**
A2

# EUROPEAN PATENT APPLICATION

(21) Application number: 88102543.1

(22) Date of filing: 22.02.88

(51) Int. Cl.⁴: **A61M 25/00**

(30) Priority: 25.02.87 IT 1947987

(43) Date of publication of application:
31.08.88 Bulletin 88/35

(84) Designated Contracting States:
AT BE CH DE ES FR GB GR LI LU NL SE

(71) Applicant: CARDIOSISTEMI S.P.A.
Via Buozzi 1/A
I-40057 Cadriano di Granarolo (Bologna)(IT)

(72) Inventor: Parravicini, Roberto
Viale Reiter, 51
Modena(IT)
Inventor: Verona, Alessandro
Via Mario Pagano, 14
Milano(IT)

(74) Representative: La Ciura, Salvatore
Via Francesco Sforza 5
I-20122 Milano(IT)

(54) A vein drain cannula.

(57) A vein drain cannula for extracorporal circulation,
of the type to be inserted into the superior vena cava
and/or the inferior vena cava during surgical opera-
tion on heart, wherein the cannula has a balloon (6)
which surrounds a portion of the cannula in prox-
imaty to one end (4) thereof, and which can be
inflated from the outside to provide for partial or total
occlusion of the lumen in a vena cava (13) thereby
to obtain partial or total cardiopulmonary bypass.

FIG.1

## A VEIN DRAIN CANNULA

The invention is relating to a vein drain cannula for extra-corporal circulation as used for incannulation of the venae cavaè during operations on heart.

When surgical operations are to be performed on heart, it is necessary that the heart is partially or totally isolated from blood flow. This is achieved by means of cannulae inserted into the superior and inferior venae cavae, through which cannulae the blood is directed to an apparatus (so-called"heart-lung machine') providing for the blood to be oxygenated and then readmitted to the patient generally by incannulation of his aorta or his femoral artery. In these cases, extra-corporal circulation is spoken of.

A method of incannulation of the venae cavae comprises the steps as follows:
-making a tobacco-pouch suture at the level of the right atrium or auricle; performing a clamping at the base of the tobacco-pouch following the location of tourniquets at the extremities of the free ends of suture; excising the atrium apex; releasing the clamp and inserting a cannula into the lumen of a vena cava as, for example, the superior vena cava, over a distance of 2-3 cm; anchoring the tourniquet under tension onto the tobacco-pouch, and commonly tying the cannula and the tourniquet rubber tube together.

A like procedure is followed for incannulation of the other vena cava, still at the level of the right atrium or auricle. The cannulae are then connected to an apparatus for extracorporal circulation.

In order that a total cardio-pulmonary bypass can be obtained by means of the vein drain cannulae as used today, a hooping of the venae cavae must be performed by the aid of ribbons that are tied around the venae cavae to hold them close to the cannulae inserted therein, or by means of ring clamps.

The clamping or hooping of a vena cava is a very delicate manipulation and many problems and drawbacks are connected therevith. In fact, the operative times become longer due to both the complex procedure in hooping the venae cavae for connection of a patient to a vein line of the cardiopulmonary bypass and the reverse procedure for disconnection.

When hooping a vena cava, a risk is at all time incurred of cutting the vena or causing injury to it, the consequences of which are well imagined.

All of the above problems become even more difficult when an operation is performed on a heart of a child, due to the small size of the concerned organ, and especially in the case of repeat operations or when complex cardiopathies are involved.

In fact, in the chest of a patient having already been subjected to heart operation, adherence phenomena are always present due to clot formation and, in the presence of adherences, isolating and hooping a vena cava are extremely complicate procedures requiring extremely long times under some circumstances.

Also, in repeat operations, the risk incurred in undergoing injuries for a vena cava having previously been subjected to hooping, is increased.

All these difficulties are overcome by a vein drain cannula for extracorporal circulation according to this invention, wherein a balloon is provided in proximity to a tip end of the vein drain cannula, which balloon can be inflated from the outside and is arranged to surround a portion of the cannula.

Formed in, and along the length of, the wall thickness of the cannula is a channel which opens at one end into the inside of the balloon and which is connected at its other end to a nonreturn valve through which air or liquid fluid can be injected for inflating the balloon.

Thus, once the cannula has been inserted into a vena cava according to a conventional procedure, it is sufficient, in order to provide a total cardiopulmonary bypass, to inflate the balloon which will occlude the lumen of the vena cava thereby causing the whole of the blood flow to be directed to the inside of the cannula.

Arranged at an intermediate location on a tube connecting the channel for injecting air into said balloon and the nonreturn valve, is a further balloon which permits the inflated state of the balloon around the cannula to be manually and visually controlled.

The use of a cannula according to this invention permits avoiding such complicate procedures for hooping a vena cava as are required today by commercially available vein drain cannulae.

Further features of the invention will more clearly appear from a reading of the following detailed description of one preferred embodiment thereof, shown by way of a non-limiting example in the accompanying drawing, wherein :

- Figure 1 is a schematic part sectional plant view, showing a cannula according to the invention when in a condition for total bypass; and

- Figure 2 is a cross-sectional view taken on the line II-II in figure 1.

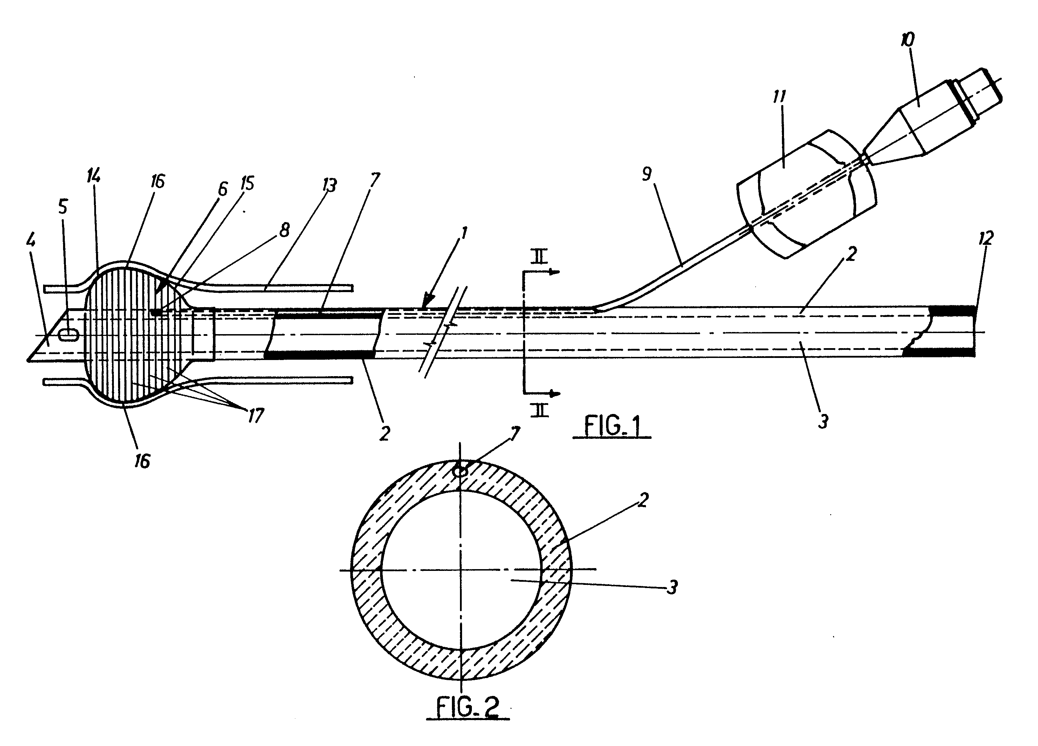

Referring now to the above figures, a vein drain cannula for extracorporal circulation according to this invention is generally designated by 1 and has a circular wall 2 and a lumen 3 whose diameter may vary as required. The thickness of wall 2 is shown in dashed lines in the non-sectional parts of figure 1 and is blackened in the sectional parts of this figure.

The extremity of the cannula which is to be inserted into a vena cava has a untraumatic flute mouth piece-shaped tip 4 and holes 5 are formed in the cannula wall 2 adjacent to this tip 4.

An expansible balloon 6 is provided in proximity to the cannula tip 4 just beyond said holes 5 on the side of the holes away from said tip 4, this balloon 6 being arranged to surround the cannula so as to extend around a given portion of its length with the two ends of the balloon being bonded or sealed to the outer surface of the cannula.

Formed in the thickness of the cannula wall 2 is a longitudinal channel 7 having an outlet port 8 opening into the inside of balloon 6. The channel 7 is connected via a small tube 9 to a nonreturn valve 10 through which air or liquid fluid can be injected as by means of a syringe, to inflate the balloon 6.

A second balloon 11 is arranged between the nonreturn valve 10 and the connecting tube 9 to permit manual or visual control of the inflated state of balloon 6.

The wall 2 of cannula 1 as well as the balloons 6 and 11 and the connecting tube 9 are preferably obtained from polyvinyl chloride (PVC) material, though other suitable materials may also be used.

The cannula may be strengthened by means of a wire as, for example, a stainless steel wire which is built in the thickness of the cannula wall 2 so as to be helically wound around this latter. In this case, it is preferred that the metal reinforcement of the cannula should terminate to a proper distance apart from the end 12 of the cannula which is away from its tip end 4 so as to maintain some flexibility in said end 12 to permit both the joining to the vein line leading to the extracorporal apparatus and the clamping of the cannula to be performed easily.

Use of the vein drain cannula according to this invention is made in conformity to a well known methodology as already described herein before.

Thus, once the cannula has been inserted into a vena cava, in order to ontain total cardiopulmonary bypass, air or liquid fluid is injected, as by a syringe, into the balloon 6 via the nonreturn valve 10 to inflate the balloon 6. The amount of inflation of balloon 6 can be determined either visually or manually as by palpation of the incannulated vena cava, or in a simpler manner, by visually or manually checking the amount of inflation of the control balloon 11.

The condition shown in figure 1 is the condition corresponding to a total cardiopulmonary bypass, wherein the whole of the blood flowing in the vena cava 13 shown schematically in the drawing, is directed to the inside of the cannula 1. The holes 5 adjacent to the cannula tip 4 will assist the blood in entering the lumen 3 of the cannula.

Advantageously, the balloon 6 has a heart-shaped profile, as viewed in figure 1, namely with the front part 14 of the balloon 6 showing a steeper slope than the rear part 15 thereof.

Such a conformation of the balloon 6 will act to form a barrier at the front to prevent blood from passing on between the balloon and the inner surface of the vena cava and, at the same time, to allow for smooth radiusing of the vena to the rear of the balloon. Furthermore, such profile of the balloon acts to prevent any dislocation of the balloon within the vena, since the pressure it exerts inside the vena will be at a greatest level at an intermediate circumference of the balloon and then decrease on both sides thereof to the terminal ends of the balloon.

The regions where the circumference of greatest pressing force contacts the inner wall of the vena cava 13 are designated by reference 16 in figure 1.

Provided over the outer surface of balloon 6 are transverse crimp formations 17 to improve tight adherence between said balloon surface and the inner surface of a vena cava.

Obviously, if no total cardiopulmonary bypass is to be obtained, the balloon 6 will be inflated only in part as required.

Deflation of balloon 6 is effected by withdrawing air or liquid **fluid** therefrom, for example, again by the aid of a syringe, through the valve means 10.

In order to test feasibility of this new methodology, experimental operations were performed on pigs 50-55 kg in weight.

The subject animals were put in extracorporal circulation by using a vein drain cannula according to the invention.

When opening the right atrium, at total cardiopulmonary bypass and cardioplegic arrest, it was possible to observe thorough tightness of the endocavial balloons, the venous flow showing to be entirely directed to the lumen in the cannulae.

As a result of this experimental work it was possible to conceive a balloon having the conformation as described herein above, capable of expanding to a greater extent in a traverse direction rather in a longitudinal direction, in order to prevent it from slippingly move within the lumen of a vena cava during intracardiac operative procedure, and to minimize its occupied space.

It should be apparent that the tip end 4 of the cannula which is shown to be in the form of a flute mouthpiece in figure 1, could take any other suitable shape, the same applying to balloon 6.

Though one particular embodiment of the invention has been described herein before and shown in the accompanying drawing, it is to be intended that many changes as to the details of construction could be made thereto without departing from the spirit and scope of the invention.

## Claims

1. A vein drain cannula for extracorporal circulation, of the type to be inserted into the superior vena cava and/or the inferior vena cava in order to provide for total or partial cardiopulmonary bypass during heart operation, characterized in that the cannula has a balloon (6) provided in proximity of the cannula end (4) intended for insertion into a vena cava, which balloon (6) can be inflated from the outside to occlude, in toto or in part, the lumen in the vena cava (13).

2. The vein drain cannula according to claim 1, wherein said balloon (6) surrounds a portion of the cannula (1) and when in inflated condition the balloon takes a heart-shaped profile, the front part (14) of the balloon which is directed towards said cannula end (4) showing a steeper slope than the rear part (15) of the balloon which is away from said cannula end (4).

3. The vein drain cannula according to claim 2, wherein the balloon (6), when in inflated state, acts with greatest pressure upon the inner wall of the vena cava (13) at the level of an intermediate circomference (16) of the balloon.

4. The vein drain cannula according to claim 3, wherein transverse crimp formations (17) are formed over the outer surface of balloon (6).

5. The vein drain cannula according to any of the preceding claims, wherein a channel (7) is formed in the thickness of wall (2) of cannula (1) and opens at one end thereof into the inside of balloon (6) via an outlet port (8), the channel (7) being connected at its other end to a nonreturn valve (10), which valve (10) permits air to be injected therethrough as by means of a syringe, to inflate the balloon (6).

6. The vein drain cannula according to claim 5, wherein a second balloon (11) is arranged on a connection between channel (7) and valve (10) and is intended for manual and visual control of the inflation state of balloon (6).

7. The vein drain cannula according to any of the preceding claims, wherein that end (4) of the cannula which is to be inserted into a vena cava (13) is cut to the shape of a flute mouthpiece.

8. The vein drain cannula according to any of the preceding claims wherein holes (5) are formed in that portion of the cannula wall (2) which extends from the cannula end (4) to the balloon (6).

9. The vein drain cannula according to any of the preceding claims, wherein the cannula is strengthened at least over a part along its length by means of a wire which is built in the thickness of the cannula wall (2) into helically wound form.

10. The vein drain cannula according to any of the preceding claims, wherein the cannula is obtained from polyvinyl chloride (PVC) material.

FIG. 1

FIG. 2

0 280 225